(19) Europäisches Patentamt
European Patent Office
Office européen des brevets

(11) EP 3 094 738 B1

(12) **FASCICULE DE BREVET EUROPEEN**

(45) Date de publication et mention de la délivrance du brevet:
**18.07.2018 Bulletin 2018/29**

(21) Numéro de dépôt: **14831025.3**

(22) Date de dépôt: **23.12.2014**

(51) Int Cl.:
*C12Q 1/04* (2006.01) *C12Q 1/44* (2006.01)

(86) Numéro de dépôt international:
**PCT/FR2014/053547**

(87) Numéro de publication internationale:
**WO 2015/097414 (02.07.2015 Gazette 2015/26)**

(54) **UTILISATION D'AU MOINS UN SUBSTRAT DE CARBOXYLESTÉRASE ET/OU DE TRIACYLGLYCÉROL-LIPASE POUR LA DÉTECTION DES BACTÉRIES DU GROUPE BACILLUS CEREUS**

VERWENDUNG VON MINDESTENS EINEM SUBSTRAT VON CARBOXYLESTERASE UND/ODER TRIACYLGLYCEROLLIPASE ZUM NACHWEIS VON BAKTERIEN DER GRUPPE BACILLUS CEREUS

USE OF AT LEAST ONE SUBSTRATE OF CARBOXYLESTERASE AND/OR TRIACYLGLYCEROL LIPASE FOR DETECTING BACTERIA OF THE GROUP BACILLUS CEREUS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.12.2013 FR 1363571**

(43) Date de publication de la demande:
**23.11.2016 Bulletin 2016/47**

(73) Titulaire: **bioMérieux**
**69280 Marcy-l'Étoile (FR)**

(72) Inventeurs:
- **CELLIER, Marie**
  **F-38390 Montalieu-Vercieu (FR)**
- **BOURGUIGNON, Marie-Pierre**
  **F-01800 Pérouges (FR)**
- **HALIMI, Diane**
  **F-01700 Saint Maurice de Beynost (FR)**

(74) Mandataire: **Murgitroyd & Company**
**Scotland House**
**165-169 Scotland Street**
**Glasgow G5 8PL (GB)**

(56) Documents cités:
**WO-A1-2010/128120 WO-A1-2011/033224**
**WO-A2-01/38559**

- **YASUO MOTOYAMA ET AL: "Rapid and sensitive detection of viable bacteria in contaminated platelet concentrates using a newly developed bioimaging system", TRANSFUSION, vol. 48, no. 11, 1 novembre 2008 (2008-11-01), pages 2364-2369, XP055144117, ISSN: 0041-1132, DOI: 10.1111/j.1537-2995.2008.01863.x cité dans la demande**
- **KIERNAN J A: "Indigogenic substrates for detection and localization of enzymes", BIOTECHNIC AND HISTOCHEMISTRY, WILLIAMS & WILKINS, BALTIMORE, MD, US, vol. 82, no. 2, 1 avril 2007 (2007-04-01), pages 73-103, XP008118888, ISSN: 1052-0295, DOI: 10.1080/10520290701375278**
- **FERENCKO L ET AL: "Esterase activity as a novel parameter of spore germination in Bacillus anthracis", BIOCHEMICAL AND BIOPHYSICAL RESEARCH COMMUNICATIONS, ACADEMIC PRESS INC. ORLANDO, FL, US, vol. 319, no. 3, 2 juillet 2004 (2004-07-02), pages 854-858, XP004512517, ISSN: 0006-291X, DOI: 10.1016/J.BBRC.2004.05.062**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 094 738 B1

## Description

### Domaine technique

La présente invention concerne le domaine du contrôle microbiologique au sens large, tel que le contrôle microbiologique d'un échantillon d'origine industrielle ou clinique. Plus particulièrement, la présente invention a trait à une méthode de détection des bactéries appartenant au groupe *Bacillus cereus* par rapport à d'autres bactéries, par détection de l'hydrolyse d'un substrat enzymatique de carboxylestérase et/ou triacylglycérol-lipase.

### Etat de la technique

Le contrôle microbiologique d'échantillons d'origines diverses (par exemple d'origine agro-alimentaire ou clinique) requiert la mise en oeuvre de techniques qui permettent la détection - par exemple aux fins d'identification et/ou de dénombrement - de micro-organismes et dont le rendu en termes de résultats doit être le plus rapide possible. D'une manière générale, lesdits micro-organismes peuvent être non pathogènes, telles les bactéries d'intérêt technologique comme les ferments ou les indicateurs de qualité ; ces derniers permettant de valider le processus de production, de matières premières aux produits bruts, jusqu'aux produits finis. Toutefois, les micro-organismes faisant l'objet d'un contrôle microbiologique sont le plus fréquemment pathogènes, nécessitant dès lors une détection rapide et précise de ceux-ci afin d'engager les actions correctives *ad hoc* le plus tôt possible. Bien évidemment, les toxines produites par de tels micro-organismes pathogènes - et responsables en tout ou partie de leur caractère pathogène - peuvent également être recherchées.

Le diagnostic clinique utilise les mêmes techniques : soit la détection et/ou le dénombrement des bactéries elles-mêmes, soit la détection des toxines.

Dans tous les cas, et quelle que soit l'origine de l'échantillon faisant l'objet d'un contrôle microbiologique, les facteurs importants pour évaluer l'efficacité de ce contrôle sont : la sensibilité, la spécificité et le temps d'obtention du résultat (« time-to-result », en langue anglaise).

Le genre *Bacillus* comporte des bactéries à Gram positif présentes dans la nature de façon ubiquitaire : dans la terre, l'eau, l'air, dans les produits alimentaires tels que les graines de céréales, le lait en poudre, les produits farineux, les épices, etc. Leur capacité à former des spores leur confère une très grande résistance dans le milieu extérieur. Les spores de *Bacillus cereus* (*B. cereus*) peuvent notamment souiller les aliments, des matières premières aux produits manufacturés. La survie desdites spores est assurée tout au long de la chaîne alimentaire. Dans des circonstances normales, *B. cereus* est présent en quantité inférieure à $10^3$ cellules par gramme d'aliment et n'a aucune incidence pathogène. Le niveau pathogène généralement admis est de l'ordre de $10^5$ cellules par gramme d'aliment. La contamination d'un individu à partir d'un aliment peut alors être responsable d'une gastro-entérite. Les gastro-entérites associées à *B. cereus* se traduisent soit par des vomissements, soit par des diarrhées. Divers aliments peuvent être incriminés : viandes, riz, plats déshydratés, sauces, soupes, etc. Des infections opportunistes à *B. cereus* peuvent aussi être observées chez des patients fragilisés tels des sujets alcooliques, immunodéprimés ou à la suite de blessures de type brûlures.

La détection (par exemple aux fins d'identification et/ou de dénombrement) des bactéries du groupe *Bacillus cereus* est donc primordiale notamment pour les laboratoires de contrôle de l'industrie agro-alimentaire ainsi que pour ceux amenés à poser des diagnostics cliniques.

De façon standard, l'isolement des bactéries du groupe *Bacillus cereus* est effectué sur des milieux de culture sélectifs conventionnels sur boîte : par exemple, les normes de type ISO (« International Organization for Standardization », en langue anglaise) ou les méthodes BAM-FDA (« Bacteriological Analytical Manual of the Food and Drug Administration », en langue anglaise) recommandent l'emploi de milieux tels que le « polymyxin egg yolk mannitol bromothymol blue Agar » (dont l'acronyme est PEMBA) ou le « mannitol-egg yolk-polymyxin Agar » (dont l'acronyme est MYP), selon leurs dénominations en langue anglaise. L'identification de *Bacillus cereus* est effectuée selon des critères morphologiques, culturaux et/ou métaboliques. Malheureusement, ces milieux PEMBA ou MYP peuvent donner lieu à des résultats faux positifs (problème de spécificité de détection) par exemple dus à un système inhibiteur peu performant ou à des résultats faux négatifs (problème de sensibilité), par exemple liés à l'absence, chez certaines souches de *B. cereus,* des caractéristiques morphologiques et/ou métaboliques clés recherchées. Enfin, certaines souches appartenant au groupe *B. cereus* présentent des réactions ambiguës, comme le décrit Fricker et al., International Journal of Food Microbiology ; 121 (2008) : 27-34.

Des milieux chromogènes en boîte ont été développés afin de tenter de réduire/supprimer les résultats faux négatifs. De tels milieux chromogènes contiennent des substrats naturels ou des substrats synthétiques chromogènes. Des activités enzymatiques spécifiques de certaines souches bactériennes sont ainsi détectées par clivage de ces substrats. Ceci a été décrit, à de nombreuses reprises, dans l'état de la technique.

D'une manière générale, la spécificité de détection peut être améliorée par l'addition, au sein du milieu de

culture, de systèmes inhibiteurs, cocktails d'antimicrobiens et/ou d'antifongiques destinés à limiter la croissance des micro-organismes non ciblés/recherchés. Toutefois, de tels cocktails d'inhibiteurs tendent généralement à retarder la croissance des micro-organismes cibles/recherchés.

Des milieux chromogènes ou fluorescents, basés sur la détection de l'activité Phosphatidylinositol-specific phospholipase C (ci-après désignée PI-PLC ; classification enzymatique : EC 4.6.1.13), ont été décrits notamment au sein des brevets US 6,284,517, EP-B-1219628 et US 6,558,917, ainsi que dans la publication de Fricker et al., 2008 susvisée. De tels milieux présentent, entre autres, l'inconvénient de donner lieu à des résultats faux négatifs (problème de sensibilité de détection), en particulier avec certaines souches de *Bacillus cereus, B. mycoides, B. weihenstephanensis* ne présentant pas d'activité PI- PLC ou une faible activité PI-PLC (*B. anthracis*), ou encore à des faux positifs (problème de spécificité de détection). En outre, le substrat fluorescent 4MU-MIP (4-methylumbelliferyl myo-inositol-1-phosphate) présente une stabilité réduite en milieu aqueux qui impose des conditions draconiennes d'utilisation, en particulier en mesure discontinue de la fluorescence, et ce, dans des conditions de pH précises, comme l'indique le brevet US 6,558,917 susvisé. Plus précisément, il est nécessaire d'effectuer la culture à pH acide puis d'alcaliniser le milieu pour augmenter la fluorescence, lue en point final.

Le brevet US 7,309,580, quant à lui, décrit un milieu en boîte combinant un substrat chromogène de phosphatidylcholine-specific phospholipase C (dont l'acronyme est « PC-PLC » ; classification enzymatique : EC 3.1.4.3) et un substrat chromogène de PI-PLC. Les couleurs respectives du premier substrat et du second substrat sont différentes et peuvent également se différencier de la troisième couleur résultant du mélange éventuel des produits de réactions enzymatiques desdits premier et second substrats.

Le milieu BCM, fourni par Biosynth® AG (Suisse), utilise un substrat chromogène de PI-PLC et un système inhibiteur de la flore bactérienne non ciblée comprenant de la polymyxine B, du triméthoprime, du sulfamethoxazole et du cycloheximide. Les performances du test sont améliorées par rapport aux milieux standards mais certaines souches atypiques peuvent rester mal identifiées (cf. Fricker et al., 2008, supra).

Des milieux chromogènes basés sur l'hydrolyse de substrats de β-glucosidase existent, tel le Brilliance™ Bacillus cereus Agar fourni par Oxoid™. Toutefois, l'emploi de tels milieux chromogènes génèrent des résultats faux-positifs avec la croissance de certaines bactéries à Gram positif exprimant une telle activité enzymatique malgré la présence d'un système inhibiteur anti-Gram positifs comprenant de la polymyxine B et du triméthoprime, ainsi que des faux négatifs (cf. Fricker et al., 2008, supra).

La demande PCT WO 2011/033224, au nom de la Demanderesse, divulgue un procédé destiné à permettre de détecter et/ou dénombrer des bactéries du groupe *Bacillus cereus* à l'aide d'un milieu réactionnel comprenant un inhibiteur de bactéries Gram négatives et un substrat fluorescent de phosphatidylcholine phospholipase C (PC-PLC). Le substrat de PC-PLC spécifiquement décrit et exemplifié dans WO 2011/033224, à savoir le 4 MU-CP (4-méthyl-umbelliferyl-choline phosphate), est un substrat qui impose de travailler en milieu liquide et qui s'avère mal adapté - voire non-adapté - à la détection et/ou au dénombrement des bactéries du groupe *Bacillus cereus* en milieu gélosé. En effet, après clivage du 4 MU-CP par la PC-PLC, la 4 MU ne demeure pas localisée au niveau de la colonie bactérienne appartenant au groupe *Bacillus cereus* mais diffuse largement au sein du milieu gélosé. La fluorescence émise est donc détectée non seulement autour de la colonie bactérienne appartenant au groupe *Bacillus cereus* mais également ailleurs. En d'autres termes, l'emploi d'un substrat fluorogène de PC-PLC, tel que le 4 MU-CP, est mal adapté à la détection et/ou au dénombrement des bactéries du groupe *Bacillus cereus* en milieu solide ou semi-solide, par exemple en milieu gélosé. En outre, d'un point de vue général, le substrat fluorogène de PC-PLC objet de WO 2011/033224 nécessite un appareillage plus ou moins sophistiqué, tel qu'une lampe à UV, afin de détecter la fluorescence induite par le clivage du substrat fluorogène sous l'effet de l'activité enzymatique recherchée.

La demande PCT WO 2010/128120 (au nom de Biosynth® AG [CH]) décrit des signalophores de type Aldol®, lesquels consistent en des dérivés d'indoxyl (1H-indolyl-3-yl) particuliers, à savoir des indoxyls conjugués sur l'amine cyclique (N-arylés). Parmi le nombre extrêmement important d'indicateurs 1*H*-Indol-3-yle testés dans WO 2010/128120 aux fins de détection de diverses souches bactériennes, le tableau IV c, présenté en pages 79 et 80 de ce document, mentionne la détection colorimétrique de souches *Bacillus cereus* par l'utilisation d'un « substrat d'estérase en C1 » (« C1-Esterase Indicator », en langue anglaise), présent dans une concentration de 0,52 mM en milieu gélosé (« Nutrient Agar »). Plus précisément, ce substrat d'estérase en C1 est le 1-(2-Benzoylphenyl)-1*H*-indol-3-yl acétate.

De manière similaire, la publication « Yasuo Motoyama et al. : "Rapid and sensitive détection of viable bacteria in contaminated platelet concentrates using a newly developed bioimaging system", TRANSFUSION, 1 novembre 2008 » divulgue l'utilisation d'un substrat d'estérase en C1, à savoir le 5(6)-carboxyfluorescein diacétate (« CFDA » ; Invitrogen, Carlsbad, CA) pour détecter plusieurs espèces de bactéries au sein de concentrés de plaquettes, parmi lesquelles *Bacillus cereus,* via la détection d'une fluorescence.

Toutefois, il s'avère que les substrats d'estérase en C1 - tels que ceux mentionnés dans WO 2010/128120 et Yasuo Motoyama et al. - sont des substrats enzymatiques peu spécifiques qui ne permettent pas, en particulier, de discriminer les bactéries du groupe *Bacillus cereus* des autres espèces de *Bacillus* fréquemment rencontrées, en particulier *Bacillus subtilis.*

Il existe donc un besoin de mettre au point une application/un procédé relativement simple (à savoir ne nécessitant pas de recourir à un appareillage sophistiqué), permettant de détecter les bactéries du groupe *Bacillus cereus,* en milieu liquide et en milieu semi-solide ou solide, non seulement avec une très bonne sensibilité de détection mais également - et surtout - avec une très bonne spécificité de détection, suffisante pour discriminer les bactéries du groupe *Bacillus cereus* des autres espèces de *Bacillus cereus* fréquemment rencontrées, en particulier *Bacillus subtilis.*

**Exposé de l'invention**

Au regard des problématiques mentionnées supra, la Demanderesse a découvert, de manière surprenante, qu'un substrat de carboxylestérase et/ou triacylglycérol-lipase de formule générale (I) (cf. infra) possédant une chaîne hydrocarbonée aliphatique X comprenant entre 11 et 17 atomes de carbone permettait notamment :

- de détecter les bactéries du groupe *Bacillus cereus* non seulement en milieu liquide mais également en milieu semi-solide ou solide, avec une sensibilité de détection et surtout une spécificité de détection optimales et un temps d'obtention du résultat (« time-to-result », en langue anglaise) tout à fait satisfaisant,
- de discriminer les bactéries du groupe *Bacillus cereus* des autres espèces de *Bacillus cereus* fréquemment rencontrées, en particulier *Bacillus subtilis,* et
- d'éviter de devoir recourir à des appareillages sophistiqués ; l'appareillage utilisé pour mettre en oeuvre le susdit substrat carboxylestérase et/ou triacylglycérol-lipase de formule générale (I) étant en effet relativement simple.

En conséquence, un objet de l'invention concerne l'utilisation d'au moins un substrat de carboxylestérase et/ou de triacylglycérol-lipase chromogène et/ou fluorogène pour la détection des bactéries du groupe *Bacillus cereus* dans un échantillon susceptible de les contenir, tel qu'un échantillon d'origine agro-alimentaire ou d'origine clinique, dans laquelle ledit substrat de carboxylestérase et/ou de triacylglycérol-lipase est un substrat de formule générale (I) :

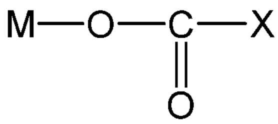
(I)

dans laquelle M représente la partie marqueur, et
X représente une chaîne hydrocarbonée aliphatique,
et dans laquelle ladite chaîne hydrocarbonée aliphatique X comprend un nombre d'atomes de carbone compris entre 11 et 17, par exemple entre 12 et 16.

Bien évidemment, l'invention concerne également le mode de réalisation dans lequel une pluralité de substrats enzymatiques différents (par exemple deux, trois, quatre, etc.) de formule générale (I) sont utilisés en combinaison.

Ainsi, les substrats de carboxylestérase et/ou triacylglycérol-lipase de formule générale (I) tels que définis précédemment permettent de détecter les bactéries du groupe *Bacillus cereus* avec une sensibilité de détection et surtout une spécificité de détection optimales et un temps d'obtention du résultat tout à fait satisfaisant, tout en ayant recours à un appareillage relativement simple. En particulier, grâce à la spécificité de détection optimale procurée par les substrats de carboxylestérase et/ou de triacylglycérol-lipase selon l'invention, il s'avère désormais possible de discriminer avec fiabilité les bactéries appartenant au groupe *Bacillus cereus* des bactéries ne faisant pas partie de ce groupe. A titre d'exemple, l'invention permet de discriminer avec fiabilité les bactéries appartenant au groupe *Bacillus cereus* de celles appartenant au groupe *Bacillus subtilis.*

De surcroît, la Demanderesse a également découvert, contre toute attente, que l'utilisation d'au moins un substrat enzymatique de formule générale (I) permettait de détecter les bactéries du groupe *Bacillus cereus* avec une sensibilité de détection et surtout une spécificité de détection optimales (permettant de discriminer avec fiabilité les bactéries appartenant au groupe *Bacillus cereus* des bactéries ne faisant pas partie de ce groupe) même lorsque l'on utilise un milieu de culture pauvre (également dénommé trivialement « milieu pauvre » ou « base pauvre »,). Par « milieu de culture pauvre » (ou « base pauvre »), l'on entend le sens communément admis en microbiologie, à savoir un milieu de culture contenant une faible concentration en éléments nutritifs. Un exemple de référence d'un tel milieu de culture pauvre est le milieu « R2A Agar » (Pharmacopée européenne ; Cat N° : 1071).

Les effets techniques avantageux susvisés sont optimaux lorsque ladite chaîne hydrocarbonée aliphatique X comprend un nombre d'atomes de carbone compris entre 13 et 15. Ceci représente donc un mode de réalisation préféré de la présente invention.

Selon un mode de réalisation préféré, la détection des bactéries du groupe *Bacillus cereus* grâce audit au moins un substrat de carboxylestérase et/ou de triacylglycérol-lipase de formule générale (I) s'opère en milieu solide ou semi-solide tel qu'un milieu gélosé.

Le susdit substrat de carboxylestérase et/ou de triacylglycérol-lipase de formule générale (I) est hydrolysé par les enzymes carboxylestérase et/ou triacylglycérol-lipase des bactéries appartenant au groupe *Bacillus cereus* selon la réaction suivante :

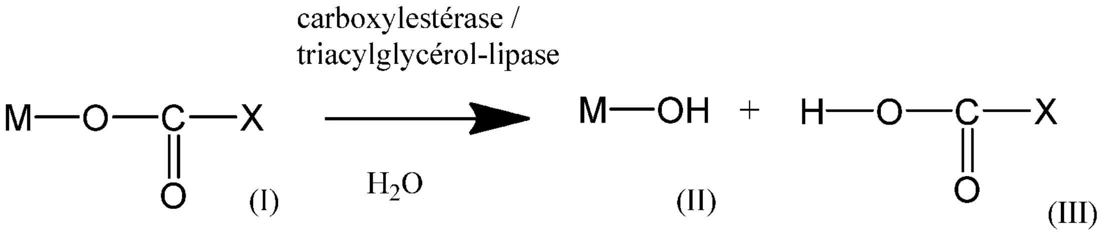


(I) (II) (III)

De préférence, X représente une chaîne hydrocarbonée aliphatique, avantageusement une chaîne hydrocarbonée aliphatique linéaire et saturée.

Selon un mode de réalisation particulièrement préféré, le substrat de carboxylestérase et/ou de triacylglycérol-lipase est choisi parmi le 5-bromo-4-chloro-3-indoxyl-myristate, le 5-bromo-4-chloro-3-indoxyl palmitate, ou une combinaison des deux.

Selon un mode de réalisation préféré, ladite partie marqueur M est :

- une partie marqueur chromogène (chromophore) sélectionnée parmi : les indoxyls (ou au moins un de leurs dérivés, tels que les Aldol®, Biosynth AG, Suisse), l'alizarine (également dénommée dihydroxyanthraquinone), l'hydroxyquinoline, le catéchol, la dihydroxyflavone, l'esculétine, le nitrophénol, le naphthol, ou au moins un de leurs dérivés ; avantageusement sélectionnée parmi les indoxyls, l'alizarine, l'hydroxyquinoline, le catéchol, la dihydroxyflavone, l'esculétine, ou au moins un de leurs dérivés ; ou
- une partie marqueur fluorogène (fluorophore) sélectionnée parmi : les dérivés de fluorescéine, de rhodamine, d'hydroxyflavone, d'ELF®97.

De préférence, M représente une partie marqueur chromogène (chromophore), de préférence sélectionnée parmi les indoxyls, l'alizarine, l'hydroxyquinoline, le catéchol, la dihydroxyflavone, l'esculétine, ou au moins un de leurs dérivés.
Selon un mode de réalisation particulièrement préféré, ladite partie marqueur chromogène est à base d'indoxyl ou d'un de ses dérivés.
Selon un mode de réalisation particulièrement préféré, ladite chaîne hydrocarbonée aliphatique X comprend 13 ou 15 atomes de carbone, de préférence 13 atomes de carbone.
Selon un mode de réalisation particulier, ledit substrat de carboxylestérase et/ou de triacylglycérol-lipase est un substrat à base d'indoxyl ou d'un de ses dérivés, ledit substrat de carboxylestérase et/ou de triacylglycérol-lipase étant utilisé en combinaison avec au moins un agent favorisant la polymérisation oxydative du dérivé indoxyl, tel qu'un complexe métallique de type citrate de fer ammoniacal. Concernant la nomenclature des substrats enzymatiques à base d'indoxyl (ou d'un de ses dérivés), il convient de comprendre les racines « indoxyl » et « indolyl » comme étant équivalentes. Ainsi, le X-myristate peut indifféremment être dénommé «5-bromo-4-chloro-3-indoxyl-myristate» ou « 5-bromo-4-chloro-3-indolyl-myristate ».
Selon un mode de réalisation préféré, ledit substrat de carboxylestérase et/ou de triacylglycérol-lipase est contenu dans un milieu réactionnel comprenant au moins un, de préférence deux et avantageusement trois des constituants suivants :

- un milieu de culture bactérien adapté aux bactéries à détecter, de préférence un milieu solide ou semi-solide tel qu'un milieu gélosé,
- un système sélectif anti Gram négatif,
- un agent antifongique.

Un autre objet de l'invention concerne un procédé de détection des bactéries du groupe *Bacillus cereus* dans un milieu solide ou semi-solide tel qu'un milieu gélosé, ledit procédé comprenant les étapes suivantes :

a) mettre en contact, au sein dudit milieu solide ou semi-solide, un échantillon susceptible de contenir des bactéries du groupe *Bacillus cereus* avec un milieu réactionnel comprenant au moins un substrat de carboxylestérase et/ou de triacylglycérol-lipase tel que défini précédemment et un inhibiteur de bactéries Gram négatives, ledit substrat étant un substrat chromogène et/ou fluorogène ;
b) incuber l'ensemble durant un laps de temps suffisant pour permettre l'apparition de colonies bactériennes du groupe *Bacillus cereus* ;

c) détecter les bactéries du groupe *Bacillus cereus* par visualisation d'une coloration et/ou d'une fluorescence due(s) à l'hydrolyse du substrat de carboxylestérase et/ou de triacylglycérol-lipase par lesdites bactéries.

Bien évidemment, en tant que substrat chromogène et/ou fluorogène, l'on utilise un substrat adapté à une utilisation en milieu solide ou semi-solide, à savoir adapté pour générer une coloration et/ou une fluorescence qui demeure(nt) localisée(s) au niveau de la zone d'hydrolyse dudit substrat par l'activité enzymatique recherchée. En effet, il est important que, contrairement à des substrats tels que le 4-MU-CP (4-méthyl-umbelliferyl-choline phosphate), le substrat chromogène et/ou fluorogène utilisé dans l'invention génère, après hydrolyse, un chromophore et/ou fluorophore qui diffuse très peu - voire ne diffuse pas - au sein du milieu solide ou semi-solide mais demeure localisé au niveau des colonies bactériennes d'intérêt (à savoir au niveau des colonies bactériennes formées par les bactéries du groupe *Bacillus cereus*).
De préférence, le milieu réactionnel comprend un milieu de culture.
Selon un mode de réalisation préféré, ledit procédé comprend une étape préalable d'enrichissement de l'échantillon.

L'invention concerne également l'utilisation d'au moins un substrat de carboxylestérase et/ou de triacylglycérol-lipase tel que défini précédemment pour la détection des bactéries du groupe *Bacillus cereus* par rapport à d'autres bactéries.

Au sens de la présente invention, les bactéries du groupe *Bacillus cereus* sont choisies parmi *Bacillus cereus, Bacillus anthracis, Bacillus thuringiensis, Bacillus mycoides, Bacillus pseudomycoides, Bacillus weihenstephanensis,* et les autres bactéries sont choisies parmi *Listeria monocytogenes, Listeria ivanovii, Staphylococcus* spp. ou les autres espèces du genre *Bacillus* spp. telles *Bacillus subtilis, Bacillus amyloliquefaciens, Bacillus atrophaeus, Bacillus licheniformis, Bacillus sphaericus, Bacillus circulans, Bacillus lentus, Bacillus pumilus, Bacillus megaterium*.

L'invention concerne également l'utilisation d'un milieu réactionnel pour la détection des bactéries du groupe *Bacillus cereus* dans un échantillon susceptible de les contenir, ledit milieu réactionnel comprenant :

- au moins un substrat de carboxylestérase et/ou de triacylglycérol-lipase tel que défini précédemment, et
- un milieu de culture bactérien adapté aux bactéries à détecter, de préférence un milieu solide ou semi-solide tel qu'un milieu gélosé.

De préférence, ledit milieu réactionnel comprend en outre au moins un système sélectif anti Gram négatif et/ou au moins un agent antifongique.

Est également décrite, au sein de la présente demande de brevet, l'utilisation d'au moins un substrat de carboxylestérase et/ou triacylglycérol-lipase chromogène et/ou fluorogène pour la détection des bactéries du groupe *Bacillus cereus* dans un échantillon susceptible de les contenir, tel qu'un échantillon d'origine alimentaire ou d'origine clinique.

Par « échantillon », l'on entend une petite partie ou petite quantité isolée d'une entité pour l'analyse. L'échantillon peut être d'origine industrielle, soit, selon une liste non exhaustive, un prélèvement d'air, un prélèvement d'eau, un prélèvement effectué sur une surface, une pièce ou un produit manufacturé, un produit d'origine alimentaire. Parmi les échantillons d'origine alimentaire, on peut citer de façon non exhaustive un échantillon de produits lactés (yaourts, fromages...), de viande, de poisson, d'oeufs, de fruits, de légumes, d'eau, de boisson (lait, jus de fruits, soda, etc.). Ces échantillons d'origine alimentaire peuvent aussi provenir de sauces ou de plats élaborés. Un échantillon alimentaire peut enfin être issu d'une alimentation destinée aux animaux, telle que notamment des farines animales. L'échantillon peut être d'origine biologique, soit animale, végétale ou humaine. Il peut alors correspondre à un prélèvement de fluide biologique (notamment sang total, sérum, plasma, urine, liquide céphalo-rachidien, lavage broncho-alvéolaire, selle, sécrétion organique), un prélèvement tissulaire ou des cellules isolées. Ce prélèvement peut être utilisé tel quel ou, préalablement à l'analyse, subir une préparation de type enrichissement, extraction, concentration, purification, selon des méthodes connues de l'homme du métier.
Le contrôle microbiologique correspond à l'analyse d'un échantillon dans le but de détecter et/ou dénombrer des micro-organismes suspectés/susceptibles d'être présents au sein dudit échantillon. Par milieu réactionnel, l'on entend un milieu comprenant tous les éléments nécessaires à la survie et/ou à la croissance des micro-organismes. Ce milieu réactionnel peut soit servir uniquement de milieu de révélation, soit servir de milieu de culture et de révélation. Dans le premier cas, la culture des micro-organismes peut être effectuée avant ensemencement, et, dans le second cas, le milieu réactionnel constitue également le milieu de culture. Le milieu réactionnel peut être solide, semi-solide ou liquide. Par milieu semi-solide ou solide, l'on entend par exemple un milieu gélifié. Préférentiellement, le milieu utilisé dans l'invention est un milieu gélifié. L'agar est l'agent gélifiant traditionnel utilisé en microbiologie pour la culture des micro-organismes, mais il est possible d'utiliser d'autres agents gélifiants comme par exemple la gelrite, la gélatine ou l'agarose. Un certain nombre de milieux gélosés sont disponibles dans le commerce et connus de l'homme du métier, comme par exemple la gélose Columbia, la gélose Trypcase-soja, la gélose Mac Conkey, la gélose Mueller Hinton ou plus généralement celles décrites dans le Handbook of Microbiological Media (CRC Press). Le milieu réactionnel peut comprendre, en outre, un ou plusieurs éléments en combinaison, tels que des facteurs de croissance comme notamment des peptones,

des hydrates de carbone, des nucléotides, des minéraux, des vitamines, du sang de mouton ou de cheval, des acides aminés, des sels, des tampons, etc. Ce milieu réactionnel peut également comprendre au moins un colorant ou indicateur de pH, pouvant être un chromophore ou un fluorophore, de préférence un chromophore. A titre d'exemple de chromophore, l'on peut citer le rouge neutre, le bleu d'aniline, le bleu de bromocresol. A titre indicatif, l'on peut citer le bleu d'Evans, le rouge neutre, le rouge de phénol, la nitroaniline, le vert malachite, le vert brillant, etc. Le milieu réactionnel peut en outre contenir un agent opacifiant tel que le dioxyde de titane, le kaolin, donnés à titre indicatif.

Ce milieu réactionnel peut se présenter sous forme de liquide, de gel prêt à l'emploi, c'est-à-dire prêt à l'ensemencement en tube, flacon ou sur boîte de Pétri. Concernant la présente invention, le milieu réactionnel contient en sus un substrat permettant la détection d'une activité enzymatique ou métabolique des micro-organismes cibles grâce à un signal détectable directement ou indirectement.

La détection des bactéries du groupe *Bacillus cereus* permet de déceler, à l'oeil nu ou à l'aide d'un appareil optique, l'existence d'une croissance des bactéries cibles (en l'espèce appartenant au groupe *Bacillus cereus*), à savoir l'apparition de colonies colorées et/ou fluorescentes (selon que l'on utilise un substrat chromogène, fluorogène ou présentant les deux caractéristiques à la fois), lesdites colonies étant colorées et/ou fluorescentes par révélation de l'activité enzymatique de type carboxylestérase et/ou de triacylglycérol-lipase des bactéries du groupe *Bacillus cereus* sur ledit substrat de carboxylestérase et/ou de triacylglycérol-lipase.

Tel qu'indiqué précédemment, la détection de la fluorescence émise après clivage des substrats enzymatiques fluorogènes impose le recours à un appareil optique, alors que la visualisation du clivage des substrats enzymatiques chromogènes peut être effectuée à l'oeil nu ou, au besoin, à l'aide d'un appareil optique. Avantageusement, la détection des bactéries du groupe *Bacillus cereus* permet également leur identification et/ou leur dénombrement.

Le dénombrement des bactéries du groupe *Bacillus cereus* consiste, quant à lui, à quantifier le nombre de colonies de bactéries du groupe *Bacillus cereus* ayant poussé sur le milieu de culture en mettant en oeuvre des techniques de microbiologie bien connues de l'homme du métier.

L'emploi d'un substrat de carboxylestérase et/ou de triacylglycérol-lipase chromogène et/ou fluorogène, de préférence chromogène, permet d'obtenir une sensibilité de détection très satisfaisante tout en conférant une très bonne spécificité : la majeure partie des bactéries Gram positif détectées sont des bactéries du groupe *Bacillus cereus.* Les autres bactéries Gram positif et les Gram négatif ne sont pas colorées et/ou fluorescentes.

En outre, un tel substrat de carboxylestérase et/ou de triacylglycérol-lipase inhibe très peu - voire pas du tout - la croissance des bactéries du groupe *Bacillus cereus* sur le milieu de culture, ce qui représente un avantage non négligeable.

Par substrat chromogène et/ou fluorogène, l'on entend un substrat permettant la détection d'une activité enzymatique ou métabolique des micro-organismes cibles/recherchés grâce à un signal détectable directement ou indirectement. Pour une détection directe, ce substrat peut être lié à une partie faisant office de marqueur, fluorogène ou chromogène (Orenga et al., 2009 ; J. Microbiol. Methods ; 79(2 :139-55)), préférablement chromogène au sens de la présente invention. Pour une détection indirecte, le milieu réactionnel peut comporter en sus un indicateur de pH, sensible à la variation de pH induite par la consommation du substrat et révélant le métabolisme des micro-organismes cibles. Ledit indicateur de pH peut être un chromophore ou un fluorophore. L'on citera, comme exemple de chromophore, le bromocresol pourpre, le bleu de bromothymol, le rouge neutre, le bleu d'aniline, le bleu de bromocresol.

Tel qu'indiqué précédemment, selon un mode de réalisation particulièrement avantageux, il est important que le substrat chromogène et/ou fluorogène utilisé dans l'invention soit adapté à une utilisation en milieu solide ou semi-solide, à savoir adapté pour générer une coloration et/ou une fluorescence qui demeure(nt) localisée(s) au niveau de la zone d'hydrolyse dudit substrat par l'activité enzymatique recherchée. En effet, il est important que, contrairement à des substrats tels que le 4 MU-CP (4-méthyl- umbelliferyl-choline phosphate), le substrat chromogène et/ou fluorogène utilisé dans l'invention ne diffuse très peu - voire ne diffuse pas - au sein du milieu solide ou semi-solide mais demeure localisé au niveau des colonies bactériennes d'intérêt (à savoir au niveau des colonies bactériennes formées par les bactéries du groupe *Bacillus cereus*).

Par « substrat de carboxylestérase et/ou de triacylglycérol-lipase », il convient de comprendre un substrat enzymatique capable, après réaction avec une enzyme de type carboxylestérase (classification enzymatique : E.C. 3.1.1.1) et/ou triacylglycérol-lipase (classification enzymatique : E.C. 3.1.1.3), de donner lieu à une réaction colorée et/ou fluorescente, selon que l'on utilise un substrat chromogène et/ou fluorogène.

Les substrats de carboxylestérase et/ou de triacylglycérol-lipase chromogènes et/ou fluorogènes utilisés selon la présente invention sont, de préférence, des substrats synthétiques, constitués en deux parties, une première partie spécifique de l'activité enzymatique à détecter, à savoir une activité enzymatique de type carboxylestérase et/ou triacylglycérol-lipase et une seconde partie faisant office de marqueur, ci-après désignée « partie marqueur ». La partie marqueur est chromogène et/ou fluorogène (chromophore et/ou fluorophore) lorsqu'elle n'est plus associée à la première partie, à savoir après clivage par l'enzyme carboxylestérase et/ou de triacylglycérol-lipase (dans le cadre d'une réaction de type hydrolyse) et séparation desdites première et deuxième parties.

Les substrats enzymatiques chromogènes (comprenant au moins un chromophore) utilisables au sens de la

présente invention peuvent être de différentes natures. Premièrement, il convient de mentionner les chromophores de type indoxyl et ses dérivés qui, en présence d'oxygène, produisent un précipité variant du bleu au rose. L'ajout d'un agent favorisant la polymérisation oxydative du dérivé indoxyl, tel qu'un complexe métallique de type citrate de fer ammoniacal dans le milieu de culture peut s'avérer avantageux. Les substrats de carboxylestérase et/ou de triacylglycérol-lipase à base d'indoxyl et ses dérivés sont particulièrement préférés du fait de leur mise en oeuvre relativement aisée et de leur bonne sensibilité de détection des bactéries cibles (notamment aux fins de l'indentification et/ou du dénombrement de ces dernières), en l'espèce de la détection des bactéries du groupe *B. cereus*. De manière générale, les substrats à base d'indoxyl (on peut citer à titre d'exemple le 5-bromo-4-chloro-3-indoxyl-beta-D-glucopyranoside ou X-beta-glucoside) sont bien connus de l'homme du métier et sont largement utilisés dans les milieux chromogènes commercialisés. Leurs applications concernent essentiellement les activités enzymatiques de type osidases, estérases, lipases et phosphatases (la phosphatase étant une activité estérase de l'acide phosphorique). Bien adaptés à une utilisation sur support solide ou semi-solide (filtre, gélose, gel d'électrophorèse, etc.), ils le sont moins à une utilisation en milieu liquide (formation d'un précipité).

Les chromophores de type Aldol® (Biosynth® AG) représentent également des chromophores d'intérêt au sens de la présente invention, dans la mesure où l'apparition d'un précipité coloré ne nécessite aucun ajout (oxygène, sels de métaux, etc.). L'utilisation de substrats enzymatiques à base d'Aldol® peut donc s'avérer particulièrement avantageuse dans le cadre d'un ensemencement dans la masse des bactéries. Ces chromophores d'Aldol® sont des dérivés d'indoxyl (1H-indolyl-3-yl) particuliers, à savoir des indoxyls conjugués sur l'amine cyclique (N-arylés), tels que divulgués dans la demande de brevet PCT publiée sous la référence WO 2010/128120 (au nom de Biosynth® AG [CH]). De tels substrats enzymatiques peuvent être obtenus auprès de la société Biosynth® AG.

Deuxièmement, il existe des chromophores tels que hydroxyquinoline, dihydroxyanthraquinone, catéchol, dihydroxyflavone ou esculétine et leurs dérivés qui, en présence de sels de fer, produisent un précipité coloré. Là encore, leurs applications concernent essentiellement des activités enzymatiques de type osidases, estérases et phosphatases.

Troisièmement, il existe des substrats enzymatiques à base de naphthol. Dans ce cas, la réaction enzyme-substrat s'effectue en deux temps, le naphthol libéré suite à l'expression de l'activité enzymatique subit un "azo-coupling" en présence d'un sel de diazonium qui est ajouté au moment de la révélation, conduisant à la formation d'un composé insoluble coloré. Ils permettent là aussi de détecter les activités osidases et estérases par l'intermédiaire du naphthol. La réaction d' "azo-coupling" s'effectue dans un milieu souvent chimiquement agressif, toxique pour les bactéries et rendant l'échantillon inutilisable pour d'autres analyses. Selon un mode de réalisation préféré de la présente invention, la partie marqueur du substrat enzymatique utilisé dans l'invention est une partie marqueur préférentiellement choisie parmi les indoxyls et leurs dérivés (3-Indoxyl, 5-Bromo-3-indoxyl, 5-Iodo-3-indoxyl, 4-Chloro-3-indoxyl, 5-Bromo-4-chloro-3-indoxyl, 5-Bromo-6-chloro-3-indoxyl, 6-Bromo-3-indoxyl, 6-Chloro-3-indoxyl, 6-Fluoro-3-indoxyl, 5-Bromo-4-chloro -3-indoxyl-N-méthyl, N-Méthyl-3-indoxyl, Aldol® ...) ; l'alizarine; l'hydroxyquinoline; le catéchol ; la dihydroxyflavone, la hydroxyflavone, le naphthol, le ELF97, l'esculétine ou l'un des dérivés. De préférence, ladite partie marqueur est une partie marqueur chromogène, avantageusement à base d'indoxyl (indolyl) ou d'un de ses dérivés.

De préférence, le substrat de carboxylestérase et/ou de triacylglycérol-lipase chromogène et/ou fluorogène, avantageusement chromogène, utilisé selon la présente invention est sélectionné parmi : 3-Indoxyl-myristate, 5-Bromo-3-indoxyl-myristate, 5-Iodo-3-indoxyl-myristate, 4-Chloro-3-indoxyl-myristate, 5-Bromo-4-chloro-3-indoxyl-myristate, 5-Bromo-6-chloro-3-indoxyl-myristate, 6-Bromo-3-indoxyl-myristate, 6-Chloro-3-indoxyl-myristate, 6-Fluoro-3-indoxyl-myristate, 5-Bromo-4-chloro -3-indoxyl-N-méthyl-myristate, N-Méthyl-3-indoxyl-myristate, Aldol®-myristate ; 3-Indoxyl-palmitate, 5-Bromo-3-indoxyl-palmitate, 5-Iodo-3-indoxyl-palmitate, 4-Chloro-3-indoxyl-palmitate, 5-Bromo-4-chloro-3-indoxyl-palmitate, 5-Bromo-6-chloro-3-indoxyl-palmitate, 6-Bromo-3-indoxyl-palmitate, 6-Chloro-3-indoxyl-palmitate, 6-Fluoro-3-indoxyl-palmitate, 5-Bromo-4-chloro-3-indoxyl-N-méthyl-palmitate, N-Méthyl-3-indoxyl-palmitate, Aldol®-palmitate, Alizarine-myristate, alizarine-palmitate, 3,4-cyclohexenoesculetine-myristate (CHE-myristate), 3,4-cyclohexenoesculetine- -palmitate (CHE-palmitate), catéchol-myristate, catéchol-palmitate, dihydroxyflavone-myristate, dihydroxyflavone-palmitate, hydroxyquinoline-myristate et hydroxyquinoline-palmitate. Avantageusement, ledit substrat est le 5-bromo-4-chloro-3-indoxyl-myristate (X-C14) ou le 5-bromo-4-chloro-3-indoxyl-palmitate (X-C16). Selon un mode de réalisation particulier, l'on peut utiliser, aux fins de la présente invention, une combinaison de 5-bromo-4-chloro-3-indoxyl-myristate (X-C14) et de 5-bromo-4-chloro-3-indoxyl-palmitate (X-C16).

Selon un mode de réalisation particulier, ledit substrat est un substrat de carboxylestérase et de triacylglycérol-lipase.

Tel qu'indiqué supra, le substrat de carboxylestérase et/ou de triacylglycérol-lipase est préférablement un substrat chromogène, clivable par l'activité carboxylestérase et/ou de triacylglycérol-lipase des bactéries du groupe *Bacillus cereus*. Ainsi, ce substrat chromogène est, de préférence, un substrat constitué d'une partie cible et d'une partie marqueur. L'hydrolyse du substrat par l'enzyme carboxylestérase et/ou de triacylglycérol-lipase des bactéries du groupe *Bacillus cereus* induit la séparation (clivage) de la partie cible et de la partie marqueur, ladite partie cible caractérisant l'activité enzymatique carboxylestérase et/ou triacylglycérol-lipase et ladite partie marqueur étant une molécule permettant de révéler la réaction d'hydrolyse via l'apparition d'une coloration au niveau du site d'hydrolyse : au niveau des

colonies.

L'homme du métier peut également utiliser une bi-boite, permettant de comparer aisément deux milieux, comprenant différents substrats ou différents mélanges sélectifs, sur lesquels on aura déposé un même échantillon biologique. Le milieu réactionnel peut comprendre un ou plusieurs agents sélectifs permettant l'inhibition de la croissance des germes Gram négatif, des levures et moisissures et des bactéries à Gram positif hormis les bactéries d'intérêt - constituées ici par les bactéries du groupe *B. cereus.*

Par « agent sélectif », l'on entend tout composé susceptible d'empêcher ou de ralentir la croissance d'un micro-organisme autre que le micro-organisme cible. A tire d'exemple, on pourra utiliser des antibiotiques, des antifongiques, du chlorure de lithium. Sans être limitatif, une concentration comprise entre 0,01 mg/l et 5 g/l en agent(s) sélectif(s) est particulièrement adaptée à la présente invention.

Concernant les systèmes sélectifs anti-Gram négatif (anti-Gram-), anti-Gram positif (anti-Gram+) et/ou antifongiques, ces derniers sont bien connus de l'homme du métier. A titre d'exemple, dans le système sélectif anti-Gram négatif utilisé aux fins de la présente invention, l'on utilise un ou plusieurs agent(s) sélectif(s) sélectionné(s) parmi l'acide nalidixique, l'aztréonam, la polymyxine B, la colistine à des concentrations connues de l'homme du métier pour obtenir l'effet escompté, à savoir l'élimination des bactéries Gram négatif.

Par antifongique (« ou agent antifongique »), l'on entend tout composé susceptible d'empêcher ou de ralentir la croissance d'une levure ou d'une moisissure. A titre indicatif, l'on peut citer notamment l'amphotéricine B, le fluconazole, l'itraconazole, le voriconazole, la cycloheximide, la 5-fluorocytosine. De préférence, l'on utilise au moins un agent antifongique à des concentrations connues de l'homme du métier pour obtenir l'effet susvisé.

Par « antibiotique », l'on entend tout composé susceptible d'empêcher ou de ralentir la croissance d'une bactérie. Les antibiotiques appartiennent notamment aux groupes des beta-lactamines, des glycopeptides, des aminosides, des polypeptides, des sulfamides, des quinolones. A titre indicatif, l'on peut citer notamment les antibiotiques cefotaxime, cefsulodine, ceftazidime, cefoxitine, ceftriaxone, cefpodoxime, aztréonam, vancomycine, gentamicine, Triméthoprime, nisine, tobramycine, moxalactam, fosfomycine, D-cylcosérine, Polymixine, Colistine, les quinolones telles que l'acide nalidixique.

Par « incuber », l'on entend porter et maintenir entre 1 et 48 heures, préférentiellement entre 4 et 24 heures, plus préférentiellement entre 16 et 24 heures, à une température appropriée, généralement comprise entre 20°C et 50°C, préférentiellement entre 30 et 40 °C.

Au sens de la présente invention, la définition de la « sensibilité de détection » est identique à celle communément admise dans l'état de la technique, à savoir la capacité à donner un résultat positif (apparition d'une réaction colorée et/ou fluorescente) lorsque la souche bactérienne cible - en l'espèce appartenant au groupe *Bacillus cereus* - est présente dans l'échantillon.

Il en va de même pour la spécificité de détection, dont la définition est conforme à celle admise dans l'état de la technique, à savoir comme la capacité à donner un résultat négatif (absence de réaction colorée et/ou fluorescente) lorsqu'une souche autre que la souche bactérienne cible - en l'espèce appartenant au groupe *Bacillus cereus* -est présente dans l'échantillon testé.

Le substrat de carboxylestérase et/ou de triacylglycérol-lipase chromogène et/ou fluorogène est utilisé à une concentration suffisante pour permettre de détecter l'apparition d'une réaction colorée et/ou fluorescente après clivage par les bactéries du groupe *Bacillus cereus.* Cette concentration est connue de l'homme du métier ou tout du moins aisément déterminable par ce dernier. A titre illustratif, la concentration en substrat de carboxylestérase et/ou de triacylglycérol-lipase chromogène et/ou fluorogène est généralement comprise entre 1 mg/L et 10 g/L, de préférence entre 5 mg/L et 6 g/L, préférablement entre 25 mg/L et 2 g/L, et avantageusement entre 25 mg/L et 500 mg/L.

**Description détaillée de l'invention**

**Exemple 1**

**1.1 Mode opératoire**

Aux fins de cet exemple, l'on utilise une base pauvre dont la composition est détaillée dans le tableau 1 présenté ci-après :

Tableau 1 : composition de la base pauvre

| Composés | Concentration en g/L |
|---|---|
| Glucose | 0,25 |
| Agar | 13 |

(suite)

| Composés | Concentration en g/L |
|---|---|
| Extrait de levure | 2 |
| Pyruvate de sodium | 0,25 |
| NaCl | 5 |
| Tampon | 0,16 |

Les différents composés de cette base pauvre sont pesés un à un. Le tout est repris dans un volume nécessaire d'eau osmosée. Les milieux sont fondus à 100°C et stérilisés par autoclavage (cycle de 121°C pendant 15 minutes).

Après retour des milieux à 55°C, les substrats enzymatiques 5-bromo-4-chloro-3-indoxyl-myristate (X-$C_{14}$) et 5-bromo-4-chloro-3-indoxyl-palmitate (X-$C_{16}$) - tous deux substrats de carboxylestérase et de triacylglycérol-lipase - sont ajoutés. Pour ce faire, des solutions mères sont préparées à 75 g/L dans un solvant organique de type DMSO. Au volume de solution mère nécessaire pour obtenir une concentration finale en substrats enzymatiques de 150 mg/L, est ajouté un volume de Tween 20.

Les milieux sont répartis en boîte de Petri stériles avant d'être ensemencés.

Le tableau 2 ci-dessous récapitule la composition en substrat enzymatique des milieux testés :

Tableau 2 : composition des milieux testés

| | Base pauvre | | |
|---|---|---|---|
| Concentration en mg/L | Milieu témoin (T) | Milieu 1 | Milieu 2 |
| X-C14 | 0 | 150 | 0 |
| X-C16 | 0 | 0 | 150 |

39 souches de *Bacillus* spp ont été testées sur chacun des trois milieux susvisés. Pour ce faire, chacun des milieux a été ensemencé à l'aide d'une oëse stérile de 10µL à partir de suspensions bactériennes calibrées à 0.5McF à l'aide d'un densitomètre ; suspensions réalisées en saline 0,85% (réf. bioMérieux 20040).

Les milieux ont ensuite été incubés pendant 48 heures à 37°C. Des lectures ont été réalisées après 24 et 48 heures d'incubation. Pour chacun de ces deux temps de lecture sont notées la croissance (nombre de cadrans et taille des colonies isolées) ainsi que les intensités de colorations obtenues (expression de l'activité enzymatique cible). Le tableau de résultats ci-dessous ne rend compte que des intensités de colorations. On note toutefois PC (« pas de croissance ») lorsqu'il y a inhibition de la croissance de la souche. Les intensités se lisent sur une échelle allant de 0 à 4 avec 0 pour une absence de coloration et 4 pour une intensité maximale de coloration.

### 1.2 Résultats

Les résultats à 24 et 48 heures d'incubation sont respectivement présentés dans les tableaux 3 et 4 infra.

Tableau 3 : résultats après 24 heures d'incubation

| Souches | N°API/QI | Milieu T | Milieu 1 | Milieu 2 |
|---|---|---|---|---|
| *B. cereus* | 02 04 010 | 0 | 1,5 | 1,5 |
| *B. cereus* | 02 04 001 | 0 | 1,5 | 1,5 |
| *B. cereus/thuringiensis* | 611 | 0 | 2,5 | 2,5 |
| *B. cereus/thuringiensis* | 691 | 0 | 2 | 2 |
| *B. cereus* | 01 06 003 | 0 | 0 | 0 |
| *B. thuringiensis* | 02 04 018 | 0 | 2,5 | 2,5 |
| *B. thuringiensis* | 02 04 012 | 0 | 2 | 1,5 |
| *B. mycoides* | 02 04 030 | 0 | 0 | 0 |
| *Bacillus weihenstephanensis* | 09 06 189 | 0 | 1 | 0,5 |

(suite)

| Souches | N°API/QI | Milieu T | Milieu 1 | Milieu 2 |
|---|---|---|---|---|
| *B. cereus/thuringiensis* | 07 06 021 | 0 | 0,5 | 0,5 |
| *B. cereus* | 11 05 179 | 0 | 0 | 0 |
| *B. mycoides* | 94 05 092 | 0 | 1 | 0,1 |
| *B. pseudomycoides* | 09 02 022 | 0 | 1,5 | 1,5 |
| *B. cereus* | 78 02 085 | 0 | 0,5 | 0 |
| *B.subtilis* | 632 | 0 | 0 | 0 |
| *B.licheniformis* | 574 | 0 | 0 | 0 |
| *B.subtilis ssp spizizenii* | 08 01 024 | 0 | 0 | 0 |
| *B.circulans* | 02 04 033 | 0 | 0 | 0 |
| *B.circulans* | 628 | 0 | 0 | 0 |
| *B. badius* | 02 04 032 | 0 | 0 | 0 |
| *B. atrophaeus* | 90 06 035 | 0 | 0 | 0 |
| *B. amyloliquefaciens* | 01 02 055 | 0 | 0 | 0 |
| *B.licheniformis* | 514 | 0 | 0 | 0 |
| *B.coagulans* | 02 04 050 | 0 | 0 | 0 |
| *B.pumilus* | 22 | 0 | 0 | 0 |
| *B.lentus* | 630 | 0 | 0 | 0 |
| *B.firmus* | 93 08 075 | 0 | 0 | 0 |
| *B. amyloliquefaciens* | 90 08 024 | 0 | 1 | 0 |
| *B.lentus* | 93 08 070 | 0 | 0 | 0 |
| *B.pumilus* | 02 04 036 | 0 | 1,5 | 1,5 |
| *B.clausii* | 98 08 107 | 0 | 0 | 0 |
| *B. vietnamensis* | 05 05 001 | PC | PC | PC |
| *B.subtilis* | 09 11 166 | 0 | 1,5 | 1 |
| *B.megaterium* | 92 06 025 | 0 | 0 | 0 |
| *B.subtilis* | 91 08 005 | 0 | 1,5 | 1 |
| *B. amyloliquefaciens* | 90 08 027 | 0 | 1,5 | 0 |
| *B.licheniformis* | 88 07 023 | 0 | 0 | 0 |
| *B.megaterium* | 87 05 008 | 0 | 0 | 0 |
| *B.firmus* | 09 07 061 | 0 | 0 | 0 |
| Sensibilité de détection *B. cereus* group (14) en % | | N/A | 78,5 | 71,5 |
| Spécificité de détection (25) en % | | N/A | 80 | 88 |
| Légende : PC = pas de croissance<br>0 = croissance, pas de coloration<br>1 = intensité de coloration faible<br>2 = intensité de coloration moyenne<br>3 = intensité de coloration forte<br>4 = intensité de coloration maximale | | | | |

Tableau 4 : résultats après 48 heures d'incubation

| Souches | N°API/QI | Milieu T | Milieu 1 | Milieu 2 |
|---|---|---|---|---|
| *B.cereus* | 02 04 010 | 0 | 2 | 2 |
| *B.cereus* | 02 04 001 | 0 | 2,5 | 2,5 |
| *B.cereus/thuringiensis* | 611 | 0 | 2,5 | 2,5 |
| *B.cereus/thuringiensis* | 691 | 0 | 2 | 2 |
| *B.cereus* | 01 06 003 | 0 | 2,5 | 2,5 |
| *B.thuringiensis* | 02 04 018 | 0 | 2,5 | 2,5 |
| *B.thuringiensis* | 02 04 012 | 0 | 2 | 2 |
| *B.mycoides* | 02 04 030 | 0 | 2 | 2 |
| *Bacillus weihenstephanensis* | 09 06 189 | 0 | 2,5 | 2,5 |
| *B.cereus/thuringiensis* | 07 06 021 | 0 | 1,5 | 1,5 |
| *B.cereus* | 11 05 179 | 0 | 1 | 0,5 |
| *B.mycoides* | 94 05 092 | 0 | 2 | 1,5 |
| *B.pseudomycoides* | 09 02 022 | 0 | 2 | 2 |
| *B.cereus* | 78 02 085 | 0 | 1,5 | 1 |
| *B.subtilis* | 632 | 0 | 0 | 0 |
| *B.licheniformis* | 574 | 0 | 0 | 0 |
| *B.subtilis ssp spizizenii* | 08 01 024 | 0 | 0 | 0 |
| *B.circulans* | 02 04 033 | 0 | 0 | 0 |
| *B.circulans* | 628 | 0 | 0 | 0 |
| *B.badius* | 02 04 032 | 0 | 0 | 0 |
| *B.atrophaeus* | 90 06 035 | 0 | 0 | 0 |
| *B.amyloliquefaciens* | 01 02 055 | 0 | 0 | 0 |
| *B.licheniformis* | 514 | 0 | 0 | 0 |
| *B.coagulans* | 02 04 050 | 0 | 0 | 0 |
| *B.pumilus* | 22 | 0 | 0 | 0 |
| *B.lentus* | 630 | 0 | 0 | 0 |
| *B.firmus* | 93 08 075 | 0 | 0 | 0 |
| *B.amyloliquefaciens* | 90 08 024 | 0 | 1,5 | 0,5 |
| *B.lentus* | 93 08 070 | 0 | 0 | 0 |
| *B.pumilus* | 02 04 036 | 0 | 2 | 1,5 |
| *B.clausii* | 98 08 107 | 0 | 2 | 1,5 |
| *B.vietnamensis* | 05 05 001 | 0 | 0 | 0 |
| *B.subtilis* | 09 11 166 | 0 | 2 | 1,5 |
| *B.megaterium* | 92 06 025 | 0 | 0 | 0 |
| *B.subtilis* | 91 08 005 | 0 | 2 | 1,5 |
| *B.amyloliquefaciens* | 90 08 027 | 0 | 1,5 | 0 |
| *B.licheniformis* | 88 07 023 | 0 | 0 | 0 |

(suite)

| Souches | N°API/QI | Milieu T | Milieu 1 | Milieu 2 |
|---|---|---|---|---|
| *B.megaterium* | 87 05 008 | 0 | 0 | 0 |
| *B.firmus* | 09 07 061 | 0 | 0,5 | 0 |
| Sensibilité de détection *B.cereus* group (14) en % | | N/A | 100 | 100 |
| Spécificité de détection (25) en % | | N/A | 72 | 80 |
| Légende : PC = pas de croissance<br>0 = croissance, pas de coloration<br>1 = intensité de coloration faible<br>2 = intensité de coloration moyenne<br>3 = intensité de coloration forte<br>4 = intensité de coloration maximale | | | | |

Aux fins de la détermination de la sensibilité et de la spécificité de détection, une souche bactérienne est considérée comme « détectée » lorsque l'intensité de coloration relevée est supérieure ou égale à 0,5, en appliquant l'échelle de détermination de l'intensité de coloration susvisée.

La sensibilité de détection (en %) est définie comme suit :

(nombre de bactéries du groupe *Bacillus cereus* « détectées » / nombre total de bactéries du groupe *Bacillus cereus* testée) x 100.

La spécificité de détection (en %) est définie comme suit :

((nombre total de bactéries non-*Bacillus cereus* – nombre de bactéries non-*Bacillus cereus* « détectées »)/ nombre total de bactéries non-*Bacillus cereus* testées) x 100.

Par « bactéries non-*Bacillus cereus* », l'on entend des bactéries qui n'appartiennent pas au groupe *Bacillus cereus.*

**1.3 Conclusion**

L'utilisation des substrats de carboxylestérase et de triacylglycérol-lipase X-C14 et X-C16 (dans les conditions mises en oeuvre) permet de détecter les souches du groupe *Bacillus cereus* avec une sensibilité de détection élevée (et des intensités de détection élevées également) - en particulier après 48h d'incubation - tout en autorisant une spécificité de détection très satisfaisante. Ceci est d'autant plus surprenant que cette détection intervient en « base pauvre », a priori moins propice à la croissance des bactéries du groupe *Bacillus cereus.*

Il ressort clairement de cette expérience la possibilité de discriminer le groupe des *Bacillus cereus* des autres espèces de *Bacillus* fréquemment rencontrées, en particulier *Bacillus subtilis,* grâce aux substrats $X\text{-}C_{14}$ et $X\text{-}C_{16}$.

**Exemple 2**

**2.1 Mode opératoire**

Dans la base pauvre autoclavée de l'exemple 1 (dont la composition est présentée au sein du tableau 1 susvisé), ramenée à 55°C, différents substrats chromogènes ou fluorogènes ont été testés avec différents tensioactifs (surfactants). 31 souches bactériennes (du genre *Bacillus*) ont été ensemencées en spot test, à raison de 1µL à partir de suspensions à 0,5 MacFarland calibrées à l'aide d'un densitomètre. Les boîtes ont ensuite été incubées en aérobie, pendant 24 heures, à 30-35°C.

Les substrats enzymatiques chromogènes 5-bromo-4-chloro-3-indoxyl-myristate ($X\text{-}C_{14}$) et 5-bromo-4-chloro-3-indoxyl-palmitate ($X\text{-}C_{16}$) ont été utilisés de façon à obtenir des concentrations finales en substrats enzymatiques de 0,15 g/L, et ce à partir de solutions mère à 50 g/L.

Les tensioactifs suivants ont été utilisés :

- Tween 20 6 g/L, et
- Triton X305 : 3 et 6 g/L

**2.2 Résultats**

Les résultats ainsi obtenus sont présentés ci-après, au sein du tableau 5.

Tableau 5 : Résultats

<table>
<tr><th rowspan="2">Milieux</th><th>Fertilité souches du groupe B. cereus (17 souches)</th><th>Sensibilité groupe B.cereus (en %)</th><th>Fertilité souches du groupe B.subtilis (5 souches)</th><th>Fertilité Bacillus sp autres (9 souches)</th><th>Spécificité (en %) (14 souches)</th></tr>
<tr><th colspan="2"></th><th></th><th></th><th></th></tr>
<tr><td>Base pauvre (témoin de croissance)</td><td>100%</td><td rowspan="4">N/A</td><td rowspan="4">N/A</td><td>78%</td><td rowspan="4">N/A</td></tr>
<tr><td>Base pauvre + MPLD + TWEEN 20</td><td>94%</td><td>78%</td></tr>
<tr><td>Base pauvre + MPLD + Triton X305 3 g/L</td><td>82%</td><td>78%</td></tr>
<tr><td>Base pauvre + MPLD + Triton X305 6 g/L</td><td>82%</td><td>78%</td></tr>
<tr><td>X-C14 / Tween 20 6 g/L</td><td>94%</td><td>88%</td><td>100%</td><td>78%</td><td>93%</td></tr>
<tr><td>X-C14 / Triton X305 3 g/L</td><td>88%</td><td>100%</td><td>100%</td><td>89%</td><td>86%</td></tr>
<tr><td>X-C14 / Triton X305 6 g/L</td><td>82%</td><td>100%</td><td>100%</td><td>78%</td><td>93%</td></tr>
<tr><td>X-C16 / Triton X305 3 g/L</td><td>88%</td><td>100%</td><td>100%</td><td>89%</td><td>93%</td></tr>
<tr><td>X-C16 / Triton X305 6 g/L</td><td>94%</td><td>100%</td><td>100%</td><td>56%</td><td>93%</td></tr>
<tr><td colspan="6">MPLD : 1-méthyl-2-pyrrolidinone</td></tr>
</table>

L'utilisation d'un substrat à base d'indoxyl contenant 14 ou 16 atomes de carbone permet d'obtenir une discrimination optimale entre les bactéries du groupe *B. cereus* et celles n'appartenant pas à celui-ci, en particulier entre les deux groupes principaux que sont *B. cereus* et *B. subtilis.*

**2.3 Conclusion :**

Une discrimination est possible entre les bactéries du groupe *B. cereus* et celles n'appartenant pas à celui-ci - en particulier entre les 2 groupes principaux que sont *B. cereus* et *B. subtilis* - avec les substrats enzymatiques fluorogènes ou chromogènes utilisés. Cette discrimination s'avère optimale lorsque le substrat enzymatique utilisé est un substrat à base d'indoxyl contenant 14 ou 16 atomes de carbone.

## Exemple 3

### 3.1 Mode opératoire

Dans la base pauvre autoclavée de l'exemple 1 (dont la composition est présentée au sein du tableau 1 susvisé), ramenée à une température de 55°C, des substrats d'estérases (carboxylestérase) à base d'alizarine de chaînes carbonées allant de 12 à 16 atomes de carbone ont été testés. Les solutions mères des substrats ont été réalisées à des concentrations de 12,5, 25 et 50 g/L solubilisées en solvant organique type DMSO en mélange avec un surfactant de type Tween et ajoutées aux milieux de sorte que la concentration finale en substrats soit respectivement 50, 100 et 200 mg/L, avec toujours la même quantité apportée aux milieux finaux de solvant et de surfactant.

Les souches bactériennes ont été ensemencées en spot test, à raison de 1µL à partir de suspensions à 0,5 Mac Farland calibrées à l'aide d'un densitomètre. Les boîtes ont ensuite été incubées en aérobie, pendant 24 heures, à 30-35°C.

### 3.2 Résultats

Les résultats ainsi obtenus sont présentés ci-après, au sein du tableau 6.

Tableau 6 : Résultats

<table>
<tr><th rowspan="2">Milieux</th><th>Fertilité groupe B.cereus (17 souches)</th><th>Sensibilité groupe B.cereus (en %)</th><th>Fertilité groupe B.subtilis (5 souches)</th><th>Fertilité Bacillus sp autres (9 souches)</th><th>Spécificité (en %) (14 souches)</th></tr>
<tr><th colspan="2"></th><th></th><th></th><th></th></tr>
<tr><td>Base pauvre témoin de croissance</td><td>100%</td><td rowspan="2">N/A</td><td rowspan="2">N/A</td><td>78%</td><td rowspan="2">N/A</td></tr>
<tr><td>Base pauvre + MPLD + Tween 20</td><td>94%</td><td>78%</td></tr>
<tr><td>Alizarine-C12 / Tween 20 6 g/L</td><td>88%</td><td>88%</td><td>100%</td><td>67%</td><td>64%</td></tr>
<tr><td>Alizarine-C14 / Tween 20 6 g/L</td><td>88%</td><td>94%</td><td>100%</td><td>78%</td><td>79%</td></tr>
<tr><td>Alizarine-C16 / Tween 20 6 g/L</td><td>94%</td><td>100%</td><td>100%</td><td>78%</td><td>64%</td></tr>
</table>

Aux fins de la détermination de la sensibilité et de la spécificité de détection, une souche bactérienne est considérée comme « détectée » lorsque l'intensité de coloration relevée est supérieure ou égale à 0,5, en appliquant l'échelle de détermination de l'intensité de coloration présentée au point 1.2 supra.

La sensibilité et la spécificité de détection sont également déterminées comme détaillé au point 1.2 supra.

### 3.3 Conclusion

Les substrats à base d'alizarine permettent de discriminer les bactéries du groupe *B. cereus* des bactéries n'appartenant pas à ce groupe avec une très bonne sensibilité et une spécificité satisfaisante (supérieure à 60%), la spécificité la plus importante étant atteinte avec le substrat enzymatique «Alizarine-C14», comportant 14 atomes de carbone.

## Revendications

1. Utilisation d'au moins un substrat de carboxylestérase et/ou de triacylglycérol-lipase chromogène et/ou fluorogène pour la détection des bactéries du groupe *Bacillus cereus* dans un échantillon susceptible de les contenir, tel qu'un échantillon d'origine agro-alimentaire ou d'origine clinique, dans laquelle ledit substrat de carboxylestérase et/ou

de triacylglycérol-lipase est un substrat de formule générale (I) :

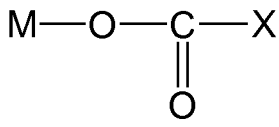

(I)

dans laquelle M représente la partie marqueur, et
X représente une chaîne hydrocarbonée aliphatique,
et dans laquelle ladite chaîne hydrocarbonée aliphatique X comprend un nombre d'atomes de carbone compris entre 11 et 17.

**2.** Utilisation selon la revendication 1, dans laquelle X représente une chaîne hydrocarbonée aliphatique linéaire.

**3.** Utilisation selon la revendication 1 ou 2, dans laquelle X représente une chaîne hydrocarbonée aliphatique linéaire et saturée.

**4.** Utilisation selon l'une des revendications 1 à 3, dans laquelle la partie marqueur M est :

- une partie marqueur chromogène sélectionnée parmi : les indoxyls, l'alizarine, l'hydroxyquinoline, le catéchol, la dihydroxyflavone, l'esculétine, le nitrophénol, le naphthol, ou au moins un de leurs dérivés ; avantageusement sélectionnée parmi les indoxyls, l'alizarine, l'hydroxyquinoline, le catéchol, la dihydroxyflavone, l'esculétine, ou au moins un de leurs dérivés ; ou
- une partie marqueur fluorogène sélectionnée parmi : les dérivés de fluorescéine, de rhodamine, d'hydroxyflavone, d'ELF®97.

**5.** Utilisation selon l'une des revendications 1 à 4, dans laquelle M représente une partie marqueur chromogène, de préférence sélectionnée parmi les indoxyls, l'alizarine, l'hydroxyquinoline, le catéchol, la dihydroxyflavone, l'esculétine, ou au moins un de leurs dérivés, avantageusement à base d'indoxyl ou d'un de ses dérivés.

**6.** Utilisation selon l'une des revendications 1 à 5, dans laquelle ladite chaîne hydrocarbonée aliphatique X comprend un nombre d'atomes de carbone compris entre 13 et 15.

**7.** Utilisation selon l'une des revendications 1 à 6, dans laquelle ladite chaîne hydrocarbonée aliphatique X comprend 13 ou 15 atomes de carbone, de préférence 13 atomes de carbone.

**8.** Utilisation selon l'une des revendications 1 à 7, dans laquelle ledit substrat de carboxylestérase et/ou de triacylglycérol-lipase est un substrat à base d'indoxyl ou d'un de ses dérivés, ledit substrat de carboxylestérase et/ou de triacylglycérol-lipase étant utilisé en combinaison avec au moins un agent favorisant la polymérisation oxydative du dérivé indoxyl, tel qu'un complexe métallique de type citrate de fer ammoniacal.

**9.** Utilisation selon l'une des revendications 1 à 8, dans laquelle ledit substrat de carboxylestérase et/ou de triacylglycérol-lipase est contenu dans un milieu réactionnel comprenant au moins un, de préférence deux et avantageusement trois des constituants suivants :

- un milieu de culture bactérien adapté aux bactéries à détecter, de préférence un milieu solide ou semi-solide tel qu'un milieu gélosé,
- un système sélectif anti Gram négatif,
- un agent antifongique.

**10.** Procédé de détection des bactéries du groupe *Bacillus cereus* dans un milieu solide ou semi-solide tel qu'un milieu gélosé, ledit procédé comprenant les étapes suivantes :

a) mettre en contact, au sein dudit milieu solide ou semi-solide, un échantillon susceptible de contenir des bactéries du groupe *Bacillus cereus* avec un milieu réactionnel comprenant au moins un substrat de carboxylestérase et/ou de triacylglycérol-lipase tel que défini dans l'une des revendications 1 à 8 et un inhibiteur de bactéries Gram négatives ;
b) incuber l'ensemble durant un laps de temps suffisant pour permettre l'apparition de colonies bactériennes

du groupe *Bacillus cereus* ;

c) détecter les bactéries du groupe *Bacillus cereus* par visualisation d'une coloration et/ou d'une fluorescence due(s) à l'hydrolyse du substrat de carboxylestérase et/ou de triacylglycérol-lipase par lesdites bactéries.

**11.** Procédé selon la revendication 10, dans lequel le milieu réactionnel comprend un milieu de culture.

**12.** Procédé selon la revendication 10 ou 11, ledit procédé comprenant une étape préalable d'enrichissement de l'échantillon.

**13.** Utilisation selon l'une des revendications 1 à 9, ou procédé selon l'une des revendications 10 à 12, pour la détection des bactéries du groupe *Bacillus cereus* par rapport à d'autres bactéries, lesdites bactéries du groupe *Bacillus cereus* étant choisies parmi *Bacillus cereus, Bacillus anthracis, Bacillus thuringiensis, Bacillus mycoides, Bacillus pseudomycoides, Bacillus weihenstephanensis,* et les autres bactéries étant choisies parmi *Listeria monocytogenes, Listeria ivanovii, Staphylococcus* spp. ou les autres espèces du genre *Bacillus* spp. telles *Bacillus subtilis, Bacillus amyloliquefaciens, Bacillus atrophaeus, Bacillus licheniformis, Bacillus sphaericus, Bacillus circulans, Bacillus lentus, Bacillus pumilus, Bacillus megaterium.*

**14.** Utilisation d'un milieu réactionnel pour la détection des bactéries du groupe *Bacillus cereus* dans un échantillon susceptible de les contenir, ledit milieu réactionnel comprenant :

- au moins un substrat de carboxylestérase et/ou de triacylglycérol-lipase tel que défini dans l'une des revendications 1 à 8, et
- un milieu de culture bactérien adapté aux bactéries à détecter, de préférence un milieu solide ou semi-solide tel qu'un milieu gélosé.

**15.** Utilisation selon la revendication 14, ledit milieu réactionnel comprenant en outre au moins un système sélectif anti Gram négatif et/ou au moins un agent antifongique.

## Patentansprüche

**1.** Eine Verwendung von mindestens einem chromogenen und/oder fluorogenen Carboxylesterase- und/oder Triacylglycerollipase-Substrat zum Nachweis von Bakterien der Gruppe *Bacillus cereus* in einer Probe, die sie enthalten kann, wie etwa einer Probe aus Lebensmitteln oder klinischen Ursprungs, wobei das Carboxylesterase- und/oder Triacylglycerollipase-Substrat ein Substrat mit der folgenden allgemeinen Formel (I) ist:

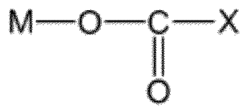

(I)

wobei M den Markerteil darstellt und
X eine aliphatische Kohlenwasserstoffkette darstellt,
und wobei die aliphatische Kohlenwasserstoffkette X eine Anzahl von Kohlenstoffatomen zwischen 11 und 17 beinhaltet.

**2.** Verwendung gemäß Anspruch 1, wobei X eine lineare aliphatische Kohlenwasserstoffkette darstellt.

**3.** Verwendung gemäß Anspruch 1 oder 2, wobei X eine lineare und gesättigte aliphatische Kohlenwasserstoffkette darstellt.

**4.** Verwendung gemäß einem der Ansprüche 1 bis 3, wobei der Markerteil M Folgendes ist:

- ein chromogener Markerteil, der ausgewählt ist aus Folgendem: Indoxylen, Alizarin, Hydroxychinolin, Catechin, Dihydroxyflavon, Esculetin, Nitrophenol, Naphthol oder mindestens einem der Derivate davon; vorzugsweise ausgewählt aus Indoxylen, Alizarin, Hydroxychinolin, Catechin, Dihydroxyflavon, Esculetin oder mindestens einem der Derivate davon; oder
- ein fluorogener Markerteil, der ausgewählt ist aus Folgendem: Derivaten von Fluorescein, Rhodamin, Hydroxyflavon, ELF®97.

**5.** Verwendung gemäß einem der Ansprüche 1 bis 4, wobei M einen chromogenen Markerteil darstellt, der vorzugsweise ausgewählt ist aus Indoxylen, Alizarin, Hydroxychinolin, Catechin, Dihydroxyflavon, Esculetin oder mindestens einem der Derivate davon, vorzugsweise auf Basis von Indoxyl oder einem der Derivate davon.

**6.** Verwendung gemäß einem der Ansprüche 1 bis 5, wobei die aliphatische Kohlenwasserstoffkette X eine Anzahl von Kohlenstoffatomen zwischen 13 und 15 beinhaltet.

**7.** Verwendung gemäß einem der Ansprüche 1 bis 6, wobei die aliphatische Kohlenwasserstoffkette X 13 oder 15 Kohlenstoffatome, vorzugsweise 13 Kohlenstoffatome, beinhaltet.

**8.** Verwendung gemäß einem der Ansprüche 1 bis 7, wobei das Carboxylesterase- und/oder Triacylglycerollipase-Substrat ein Substrat auf Basis von Indoxyl oder einem Derivat davon ist, wobei das Carboxylesterase- und/oder Triacylglycerollipase-Substrat in Kombination mit mindestens einem Mittel zur Förderung der oxidativen Polymerisation des Indoxylderivats, wie etwa einem Metallkomplex der Art ammoniakalisches Eisenzitrat, verwendet wird.

**9.** Verwendung gemäß einem der Ansprüche 1 bis 8, wobei das Carboxylesterase- und/oder Triacylglycerollipase-Substrat in einem Reaktionsmedium enthalten ist, das mindestens einen, vorzugsweise zwei und vorzugsweise drei der nachfolgenden Bestandteile beinhaltet:

- ein bakterielles Kulturmedium, das an die nachzuweisenden Bakterien angepasst ist, vorzugsweise ein festes oder halbfestes Medium, wie ein Agarmedium,
- ein selektives Anti-gramnegativ-System,
- ein fungizides Mittel.

**10.** Ein Verfahren zum Nachweis von Bakterien der Gruppe *Bacillus cereus* in einem festen oder halbfesten Medium, wie einem Agarmedium, wobei das Verfahren die folgenden Schritte beinhaltet:

a) In-Kontakt-Bringen, in dem festen oder halbfesten Medium, einer Probe, die Bakterien der Gruppe *Bacillus cereus* enthalten kann, mit einem Reaktionsmedium, das mindestens ein Carboxylesterase- und/oder Triacylglycerollipase-Substrat wie in einem der Ansprüche 1 bis 8 definiert und einen Inhibitor von gramnegativen Bakterien beinhaltet;
b) Inkubieren des Ganzen für eine ausreichende Zeitperiode, um das Auftreten von Bakterienkolonien der Gruppe *Bacillus cereus* zu ermöglichen;
c) Nachweisen der Bakterien der Gruppe *Bacillus cereus* durch Sichtbarmachung einer Verfärbung und/oder einer Fluoreszenz aufgrund der Hydrolyse des Carboxylesterase- und/oder Triacylglycerollipase-Substrats durch die Bakterien.

**11.** Verfahren gemäß Anspruch 10, wobei das Reaktionsmedium ein Kulturmedium beinhaltet.

**12.** Verfahren gemäß Anspruch 10 oder 11, wobei das Verfahren einen vorhergehenden Schritt des Anreicherns der Probe beinhaltet.

**13.** Verwendung gemäß einem der Ansprüche 1 bis 9 oder Verfahren gemäß einem der Ansprüche 10 bis 12 zum Nachweis von Bakterien der Gruppe *Bacillus cereus* im Vergleich zu anderen Bakterien, wobei die Bakterien der Gruppe *Bacillus cereus* ausgewählt sind aus *Bacillus cereus, Bacillus anthracis, Bacillus thuringiensis, Bacillus mycoides, Bacillus pseudomycoides, Bacillus weihenstephanensis* und die anderen Bakterien ausgewählt sind aus *Listeria monocytogenes, Listeria ivanovii, Staphylococcus* spp. oder den anderen Arten der Gattung *Bacillus* spp., wie etwa *Bacillus subtilis, Bacillus amyloliquefaciens, Bacillus atrophaeus, Bacillus licheniformis, Bacillus sphaericus, Bacillus circulans, Bacillus lentus, Bacillus pumilus, Bacillus megaterium.*

**14.** Eine Verwendung eines Reaktionsmediums zum Nachweis von Bakterien der Gruppe *Bacillus cereus* in einer Probe, die diese enthalten kann, wobei das Reaktionsmedium Folgendes beinhaltet:

- mindestens ein Carboxylesterase- und/oder Triacylglycerollipase-Substrat wie in einem der Ansprüche 1 bis 8 definiert und
- ein bakterielles Kulturmedium, das an die nachzuweisenden Bakterien angepasst ist, vorzugsweise ein festes

oder halbfestes Medium, wie ein Agarmedium.

**15.** Verwendung gemäß Anspruch 14, wobei das Reaktionsmedium ferner mindestens ein selektives Anti-gramnegativ-System und/oder mindestens ein Fungizid beinhaltet.

**Claims**

**1.** Use of at least one chromogenic and/or fluorogenic carboxylesterase and/or triacylglycerol-lipase substrate for detecting the bacteria of the *Bacillus cereus* group in a sample capable of containing them, such as a sample of agri-food origin or clinical origin, wherein said carboxylesterase and/or triacylglycerol-lipase substrate is a substrate of general formula (I):

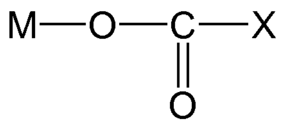

(I)

wherein M represents the labelling part, and
X represents an aliphatic hydrocarbon chain,
and wherein said aliphatic hydrocarbon chain X comprises a number of carbon atoms between 11 and 17.

**2.** The use according to claim 1, wherein X represents a linear aliphatic hydrocarbon chain.

**3.** The use according to claim 1 or 2, wherein X represents a linear and saturated aliphatic hydrocarbon chain.

**4.** The use according to one of claims 1 to 3, wherein the labelling part M is:

- a chromogenic labelling part selected from: indoxyls, alizarin, hydroxyquinoline, catechol, dihydroxyflavone, esculetin, nitrophenol, naphthol, or at least one derivative thereof; advantageously selected from indoxyls, alizarin, hydroxyquinoline, catechol, dihydroxyflavone, esculetin, or at least one derivative thereof; or
- a fluorogenic labelling part selected from: the derivatives of fluorescein, of rhodamine, of hydroxyflavone, of ELF®97.

**5.** The use according to one of claims 1 to 4, wherein M represents a chromogenic labelling part, preferably selected from indoxyls, alizarin, hydroxyquinoline, catechol, dihydroxyflavone, esculetin, or at least one derivative thereof, advantageously based on indoxyl or one derivative thereof.

**6.** The use according to one of claims 1 to 5, wherein said aliphatic hydrocarbon chain X comprises a number of carbon atoms between 13 and 15.

**7.** The use according to one of claims 1 to 6, wherein said aliphatic hydrocarbon chain X comprises 13 or 15 carbon atoms, preferably 13 carbon atoms.

**8.** The use according to one of claims 1 to 7, wherein said carboxylesterase and/or triacylglycerol-lipase substrate is a substrate based on indoxyl or one derivative thereof, said carboxylesterase and/or triacylglycerol-lipase substrate being used in combination with at least one agent which promotes the oxidative polymerisation of the indoxyl derivative, such as a metal complex of the ammonium ferric citrate type.

**9.** The use according to one of claims 1 to 8, wherein said carboxylesterase and/or triacylglycerol-lipase substrate is contained in a reaction medium comprising at least one, preferably two and advantageously three of the following components:

- a bacterial culture medium suitable for the bacteria to be detected, preferably a solid or semi-solid medium such as an agar medium,
- an anti-Gram negative selective system,
- an antifungal agent.

**10.** A method for detecting bacteria of the *Bacillus cereus* group in a solid or semi-solid medium such as an agar medium, said method comprising the following steps:

a) placing a sample capable of containing bacteria of the *Bacillus cereus* group in contact, within said solid or semi-solid medium, with a reaction medium comprising at least one carboxylesterase and/or triacylglycerol-lipase substrate such as defined in one of claims 1 to 8 and a Gram-negative bacteria inhibitor;
b) incubating the whole for a time period sufficient to enable the appearance of bacterial colonies of the *Bacillus cereus* group;
c) detecting the bacteria of the *Bacillus cereus* group through observing a coloration and/or a fluorescence caused by the hydrolysis of the carboxylesterase and/or triacylglycerol-lipase substrate by said bacteria.

**11.** The method according to claim 10, wherein the reaction medium comprises a culture medium.

**12.** The method according to claim 10 or 11, said method comprising a prior step of sample enrichment.

**13.** The use according to one of claims 1 to 9, or the method according to one of claims 10 to 12, for detecting the bacteria of the *Bacillus cereus* group as distinct from other bacteria, said bacteria of the *Bacillus cereus* group being chosen from *Bacillus cereus, Bacillus anthracis, Bacillus thuringiensis, Bacillus mycoides, Bacillus pseudomycoides, Bacillus weihenstephanensis,* and the other bacteria being chosen from *Listeria monocytogenes, Listeria ivanovii, Staphylococcus* spp. or the other species of the genus *Bacillus* spp. such as *Bacillus subtilis, Bacillus amyloliquefaciens, Bacillus atrophaeus, Bacillus licheniformis, Bacillus sphaericus, Bacillus circulans, Bacillus lentus, Bacillus pumilus, Bacillus megaterium.*

**14.** Use of a reaction medium for detecting the bacteria of the *Bacillus cereus* group in a sample capable of containing them, said reaction medium comprising:

- at least one carboxylesterase and/or triacylglycerol-lipase substrate such as defined in one of claims 1 to 8, and
- a bacterial culture medium suitable for the bacteria to be detected, preferably a solid or semi-solid medium such as an agar medium.

**15.** The use according to claim 14, said reaction medium further comprising at least one anti-Gram negative selective system and/or at least one antifungal agent.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6284517 B
- EP 1219628 B
- US 6558917 B
- US 7309580 B
- WO 2011033224 A
- WO 2010128120 A


### Littérature non-brevet citée dans la description


- **FRICKER et al.** *International Journal of Food Microbiology,* 2008, vol. 121, 27-34
- **YASUO MOTOYAMA et al.** Rapid and sensitive détection of viable bacteria in contaminated platelet concentrates using a newly developed bioimaging system. *TRANSFUSION,* 01 Novembre 2008
- Handbook of Microbiological Media. CRC Press
- **ORENGA et al.** *J. Microbiol. Methods,* 2009, vol. 79 (2), 139-55